# EUROPÄISCHE PATENTSCHRIFT

(11) **EP 1 827 272 B1**
(45) Veröffentlichungstag und Bekanntmachung des Hinweises auf die Patenterteilung: **02.04.2008**
(21) Anmeldenummer: 05848257.1
(22) Anmeldetag: 02.12.2005
(51) Int. Cl.: A61B 17/72

(54) **Intramedullärer Marknagel**
Intramedullary nail
Clou intramédullaire

(30) Priorität: 23.12.2004 DE 102004063396
(43) Veröffentlichungstag der Anmeldung: 05.09.2007
(73) Patentinhaber: Zielsdorf, Michael, 34516 Vöhl (DE)
(72) Erfinder: ZILLERT, Markus, 40627 Düsseldorf (DE)
(74) Vertreter: Rolf, Gudrun
(86) Internationale Anmeldenummer: PCT/DE2005/002186
(87) Internationale Veröffentlichungsnummer: WO 2006/066536

(56) Entgegenhaltungen:
- WO-A-97/49348
- WO-A-99/22662

## Beschreibung

Die Erfindung betrifft einen Marknagel gemäß dem Oberbegriff des Hauptanspruchs, der vom Dokument WO 97/49348 A abgeleitet wird.

Es ist eine weitere Vorrichtung zur Stabilisierung von Röhrenknochenbrüchen sowie von Gelenken bekannt DE 38 35 682 A1, die als Stabilisierungselemente ein- oder mehrgängig ineinander gewickelte Schraubenfedern aufweist, die zum Teil um eine Seele in Form eines elastisch verformbaren Drahtes oder eines Bündels von elastisch verformbaren Drähten herumgewickelt sind. Eine solche Schraubenfeder kann an den Enden mit einer Einrichtung zum Festlegen am Knochen ausgeführt sein.

Nachteilig an dieser bekannten Vorrichtung ist die Unbestimmtheit der Lage der Schraubenfedern sowie die Unmöglichkeit einer Rotationsverhinderung wie auch einer Fixierung einzelner Bruchstücke im Bereich einer Fraktur, da sich die Federeigenschaften der Schraubenfedern dort nicht behindern lassen.

Weiterhin ist ein Nagelsystem für die intramedulläre Osteosynthese bei Schaftfrakturen aller Art bekannt, DE 197 31 298 A1, welches einen rohrförmigen Außenkörper mit Durchbrüchen aufweist, aus denen sich Haken nach außen erstrecken, die durch Betätigung eines Innenkörpers nach außen geschwenkt werden können, wodurch sich die Haken an der Innenwand eines Röhrenknochens anlegen können sollen.

Dieses Nagelsystem ist extrem aufwändig und weist sehr viele Bauteile auf, kann aber prinzipbedingt nur Punktkontakte mit der Innenfläche eines Röhrenknochens erzeugen und diese auch nur in einem engen und über die Länge identischen Umfangsbereich.

Einfachere Marknägel zur Verriegelungsnagelung sind bekannt, deren Einbau während eines Eingriffs jedoch nur mittels intraoperativer Röntgendarstellung kontrolliert werden kann, was aufgrund der Dauer nicht nur für einen Patienten, sondern insbesondere für das OP-Personal äußerst belastend ist.

Des Weiteren müssen solche einfachen Marknägel mittels Querschrauben im Knochen gesichert werden, wozu insbesondere bei langen Oberschenkelknochen, aber auch bei Oberarm- und Unterschenkelknochen aufwändige Zielgeräte erforderlich sind, um solche Querschrauben unter möglichst geringer Verletzung der Haut durch diese hindurch punktgenau setzen zu können. Diese zusätzlich in eine Extremität einzubringenden Operationswunden müssen nach einer Operation aber auch nach einer operativen Entfernung der Querschrauben aufwändig versorgt werden, was zusätzliche Belastungen für einen Patienten mit sich bringt.

Aufgabe der Erfindung ist es, einen Marknagel zur Verfügung zu stellen, der einfach aufgebaut ist, sehr praxisorientiert, leicht, schnell, sicher und bei nur geringem Einsatz von intraoperativer Röntgendarstellung eingesetzt werden kann, ohne dabei Eingriffe in unversehrte Bereiche einer Extremität zu erfordern.

Die Lösung dieser Aufgabe ergibt sich in Verbindung mit den Oberbegriffsmerkmalen in erfindungsgemäßer Art und Weise aus den Merkmalen des kennzeichnenden Teils des Hauptanspruchs.

Der Marknagel, der sich insbesondere zur geschlossenen Reposition einer Fraktur eines Röhrenknochens durch Einsetzen in eine Bohrung in dessen Markhöhle eignet, ist dadurch gekennzeichnet, dass er einen Nagelkern aufweist, an dessen vorderem Bereich der Anfang eines äußeren Bandes festgelegt ist, welches um den Nagelkern herum aufgewickelt ist und dessen Ende im hinteren Bereich um den Nagelkern drehverriegelbar festgelegt ist, wobei sich bei einer Drehung entgegen seiner Aufwickelrichtung die radiale Kontur des Marknagels erweitert. Hierdurch wird vorteilhaft erreicht, dass sich die Außenseite des Bandes an der Innenseite einer Durchgangsbohrung bzw. unmittelbar an die Innenseite des Markraumes eines Röhrenknochens anlegen kann bzw. durch ein weiteres Drehen entgegen der Aufwickelrichtung eine radial wirkende Kraft erzeugt werden kann, mit der die Einzelteile eines Knochens optimal ausgerichtet und repositioniert geklemmt werden können, wodurch ein optimales Heilungsergebnis erzielt werden kann.

Der technische Aufbau eines solchen Marknagels ist extrem einfach, leicht von jedem Notfallarzt zu verstehen und dadurch auch sehr sicher in der korrekten Anwendung. Auf den Einsatz von distalen Fixierschrauben des Nagels kann hier vollständig verzichtet werden, so dass eine postoperative Schnittwundenversorgung in diesem Bereich nicht mehr erforderlich wird.

Durch die Klemmkraft des Bandes wird eine starke seitliche kortikale Abstützung und damit eine optimale Frakturstabilisierung erreicht, was des Weiteren zu einem schnelleren Ablauf der Frakturheilung führt. Frakturheilungsstörungen in Folge mangelhafter Frakturfixation können bei der Verwendung des erfinderischen Marknagels vollständig ausgeschlossen werden. Längsgespaltene Knochenbrüche oder solche, die sehr nahe an einem Gelenk vorliegen, können mit dem erfindungsgemäßen Marknagel jedoch nicht behandelt werden. Zur Entfernung des Marknagels werden die Bänder wieder stärker um den Nagelkern herum aufgewickelt, wodurch sich der Außendurchmesser des Marknagels wieder verringert, sodass er problemlos aus der Bohrung oder der Markhöhle herausgezogen werden kann.

Weitere vorteilhafte Ausführungsformen der Erfindung ergeben sich mit und in Kombination aus den nachfolgenden Unteransprüchen.

Als besonders vorteilhaft ist eine Ausführung hervorzuheben, bei der im vorderen Bereich des Marknagels der Anfang eines weiteren inneren Bandes festgelegt ist, welches unter dem äußeren Band um den Nagelkern herum aufgewickelt ist und dessen Ende ebenfalls im hinteren Bereich des Marknagels drehverriegelbar um den Nagelkern festgelegt ist. Ein solches inneres Band vergrößert bei einer Drehung entgegen seiner Aufwicklungsrichtung ebenfalls den Außendurchmesser des Marknagels bzw. legt sich mit seiner Außenseite an der Innenseite des äußeren Bandes an und stabilisiert so das Band und damit den gesamten Marknagel. Diese Konstruktion ermöglicht die Verwirklichung einer hohen Steifigkeit, die etwa der eines dünnen Rohres entsprechen kann.

Dadurch, dass das Band aus federelastischem Material besteht, etwa aus chirugischem Stahl, Titan oder körperfreundlichem Kunststoff, lassen sich die gewünschten Eigenschaften des Marknagels hinsichtlich der erforderlichen Kontaktkraft wie auch die Art und der Ablauf der Verformung, vorteilhafterweise zunächst von dem vorderen Bereich des Marknagels über die Markraumtaille bis zum sich wieder erweiternden Ende eines Markraumes optimal festlegen.

Gemäß weiterer vorteilhaften Ausführungsformen der Erfindung können auch mehrere innere oder äußere Bänder gleich- oder vor allen Dingen gegensinnig sowie ein- oder mehrgängig um den Nagelkern herum aufgewickelt sein, wobei bei einer einlagigen Version der inneren Bänder diese unmittelbar um den Kern herum aufgewickelt sind und bei einer einlagigen Ausführungsform der äußeren Bänder diese um die inneren Bänder herum aufgewickelt sind.
Dieser Variationsreichtum erlaubt es, für die häufigsten Arten von Frakturen von Knochen beliebiger Größe jeweils einen optimal angepassten Marknagel zur Verfügung stellen zu können, da die geforderte Festigkeit quasi beliebig hoch eingestellt werden kann.

Besonders vorteilhaft ist es, dass die radialen Außenflächen des inneren Bandes und die radialen Innenflächen des äußeren Bandes als gegenseitige Funktionsflächen rau ausgebildet sind, so dass die gemeinsamen Kontaktflächen der Bänder Stabilisierungspunkte eines in eine Durchgangsbohrung eines Knochens eingesetzten und verspannten Marknagels in Form einer kraft- und reibschlüssigen Verbindung erzeugen. Hierdurch wird die Stabilität des Marknagels weiter vergrößert.

Bei einer bevorzugten Ausführungsform der Erfindung sind die Bänder im unverbauten Zustand vorgespannt und um den Nagelkern im Bereich ihrer hinteren Enden gegeneinander oder gegen den Nagelkern über einen Spann- und Verschlussmechanismus verriegelt, so dass sie sich nach einer Entriegelung selbständig entgegen dem Drehsinn der Wickelung aufdrehen und so den Außendurchmesser des Marknagels entweder gleichmäßig oder aber in vorteilhafter Art und Weise so aufweiten, dass sich das äußere Band an die Innenkonturen des Markraumes eines Röhrenknochens anlegt. Eine solche Funktion kann beispielsweise dadurch erzeugt werden, dass das innere und/ oder das äußere Band in unterschiedlichen Bereichen des Nagelkerns unterschiedlich breit oder dick ausgebildet sind oder aber das das innere Band und/ oder das äußere Band in unterschiedlichen Breichen des Nagelkerns unterschiedlich stark elastisch vorgespannt ist. Denkbar ist es auch, dass beispielsweise das äußere Band über seine gesamten Länge oder zumindest teilweise, von der Nagelspitze ausgesehen etwa über die ersten 2/3 seiner Länge einen sich kontinuierlich verändernden Querschnitt, insbesondere eine zunehmende Breite aufweist, um die geforderte Funktion zu erfüllen.

Eine solche vorteilhafte Konstruktion ermöglicht es des Weiteren und auch bei nicht vorgespannten Bändern, dass das äußere Band beim Aufdrehen gegen den Drehsinn sich zunächst im Bereich des Kopfstückes des Marknagels an die Innenkontur eines Knochens anlegt und erst beim weiteren Aufdrehen zunächst im mittleren Bereich der Markraumtaille eines Röhrenknochens und anschließend im Endbereich des Marknagels an den sich wieder erweiternden Bereich des Markraumes anlegt.

Zur Sicherstellung der Funktion ist der Marknagel an seinem hinteren Ende mit einem Spann- und Verschlussmechanismus ausgestattet, der zumindest eine Innenbuchse und eine Außenbuchse aufweist, die gegenseitige Rasten oder Rastflächen wie etwa selbsthemmende Kegelsitze besitzen, wobei das innere Band an der um den Nagelkern drehbaren Innenbuchse und das äußere Band an der ebenfalls um den Nagelkern drehbaren Außenbuchse festgelegt ist.

Zum Einsetzen des Marknagels ist dieser mit einer davon lösbaren Montagehilfe ausgestattet, die Betätigungsvorrichtungen für den Spann- und Verschlussmechanismus aufweisen.

Vorteilhafterweise besitzt der Marknagel an seinem hinteren Bereich Kontaktflächen zur Anordnung eines Adapters als weitere mechanische Vorrichtung zur Stabilisierung von Knochenbrüchen, wie sie etwa für eine Fraktur eines Schenkelhalsknochens eines Oberschenkelknochens bekannt ist.

Auf dem Ende des Nagelkerns ist vorteilhafterweise ein Gewinde vorgesehen, auf dem die Innenbuchse des Spann- und Verschlussmechanismus aufgeschraubt ist, so dass gewährleistet ist, dass bei einem gegenseitigen Verrasten der Innenbuchse und der Außenbuchse eine definierte Position des Spann- und Verschlussmechanismus zum Nagelkern vorhanden ist.

Bei einer weiteren vorteilhaften Ausgestaltung des Gegenstandes der Erfindung ist der Nagelkern hohl ausgebildet und von einer Hülse und/ oder einer Welle durchtreten, so dass am vorderen Bereich des Kopfstückes des Marknagels der Anfang eines inneren und/ oder äußeren Bandes festgelegt werden kann, wobei vom hinteren Ende des Marknagels dieses über einen entsprechend ausgebildeten Spann- und Verschlussmechanismus von hinten gegen seine Drehrichtung abge-wickelt und aufgespannt werden kann.

Eine erfinderische Weiterbildung des Marknagels besitzt etwa zwei äußere Bänder von denen eines vom vorderen Bereich des Marknagels aus und eines vom hinteren Bereich des Marknagels aus aufdrehbar ist, so dass eine optimale Anpassung des Bandes an den sich von einer Markraumtaillie beidseitig trompetenhaft erweiternden Markraum und damit eine optimale seitliche kortikale Abstützung möglich wird.

Der Marknagel kann je nach Bedarf gerade oder gebogen ausgebildet oder auch entsprechend der geforderten Raumform plastisch verbiegbar sein.

Weiterhin kann der Nagelkern eines Marknagels in vorteilhafter Weise mehrteilig ausgebildet sein, sodass sich diese Teile gegeneinander verdrehen lassen. Dies erweitert die Möglichkeiten zur konstruktiven Gestaltung sehr wesentlich.

Bei einer weiteren sehr vorteilhaften Ausführungsform der Erfindung sind zwei gegeneinander aufgewickelte Bänder an Ihren vorderen Enden miteinander verbunden oder aber ein Band von vorne herein einteilig ausgebildet und von seinem vorderen Ende aus gegensinnig um den Nagelkern aufgewickelt ist, sodass der Nagelkern verkürzt ausgebildet sein kann und sich nur bis zu einem Teilbereich in Richtung des vorderen Endes des Marknagels erstreckt. Bei einer extremen Ausführungsform dieser Ausgestaltung ist der Nagelkern sogar bis auf den Spann- und Verschlussmechanismus reduziert, wodurch quasi im Einbauzustand ein stabiles starres Netzgitter als Marknagel übrig bleibt, was zu einer enormen Material- und Gewichtseinsparung führt, wie auch zu einer sehr kostengünstigen Variante.

Nachfolgend ist ein Ausführungsbeispiel der Erfindung anhand der Zeichnungen näher beschrieben. Es zeigen:
- Fig. 1: eine grobe Skizze eines Marknagels,
- Fig. 2: eine geschnittene Teilansicht eines Spann- und Verschlussmechanismus,
- Fig. 3: einen Schnitt durch eine Schlüsselverzahnung eines Spann- und Verschlussmechanismus,
- Fig. 4: einen Schnitt durch eine Innenverrastung von Innen- und Außenbuchse und
- Fig. 5: eine grobe Skizze eines Marknagels mit stark reduziertem Nagelkern.
Der Marknagel 1 besteht aus einem Nagelkern 2, um den von seinem Kopfstück 5 ein inneres Band 3 und ein äußeres Band 4 gegensinnig zum inneren Band 3 aufgewickelt ist, wobei die vorderen Enden der Bänder 3 und 4 etwa über ein Laser-Schweißverfahren am Kopfstück 5 ortsfest angeordnet sind.

Im hinteren Bereich des Marknagels 1 sind die Enden der Bänder 3 und 4 an einer Außenbuchse 8 bzw. einer Innenbuchse 9 des Spann- und Verschlussmechanismus 7 angeordnet und dort beispielsweise ebenfalls mittels eines Laser verschweißt. Die gegensinnig aufgewickelten Bänder 3 und 4 überdecken sich an Kontaktflächen 6, wobei die zueinander gerichteten Oberflächen der Bänder 3 und 4 so ausgebildet sind, dass dort eine kraft- und reibschlüssige Fixierung erfolgt, die den gesamten Marknagel 1 stabilisiert.

Die Außenbuchse 8 und die Innenbuchse 9 weisen gegenseitige Rastungen auf und sind über ein Gewinde 10 auf dem Ende des Nagelkerns 2 und in der Innenbuchse 9 auf dieser verdrehbar festgelegt.

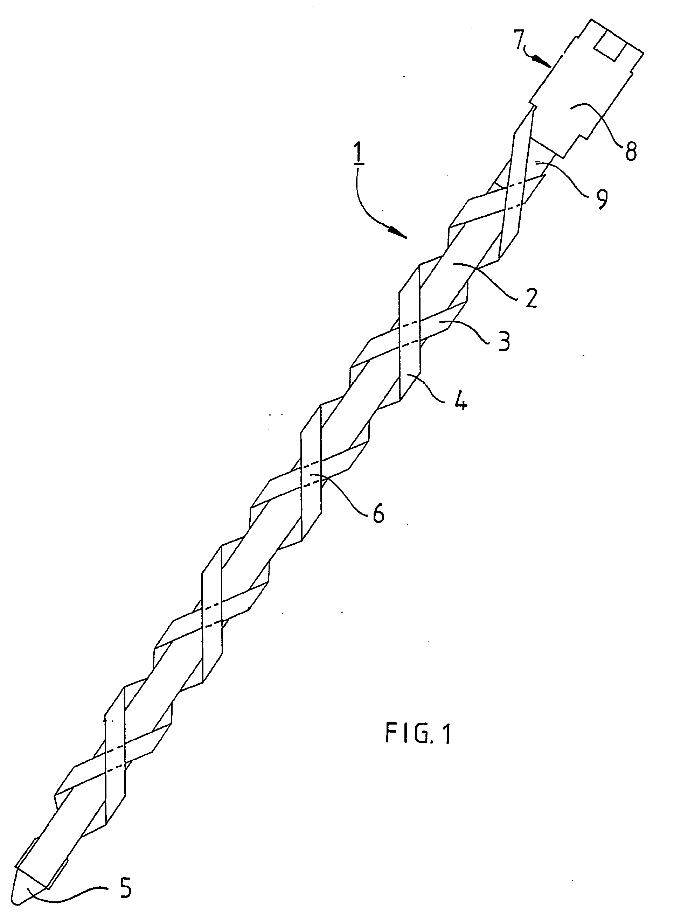

Die in Fig. 1 nur beispielhaft dargestellte Ausführungsform weist in einer praxisorientierten Ausführungsform eine deutlich höhere Anzahl von Windungen des inneren Bandes 3 wie auch des äußeren Bandes 4 auf, so dass sich eine erhebliche höhere Anzahl an gemeinsamen Kontaktflächen 6 zwischen den Bändern 3 und 4 ergibt und damit eine extrem vergrößerte Steifigkeit des Marknagels 1 und eine damit verbundene optimierte Fixierung einer Fraktur vorhanden ist.

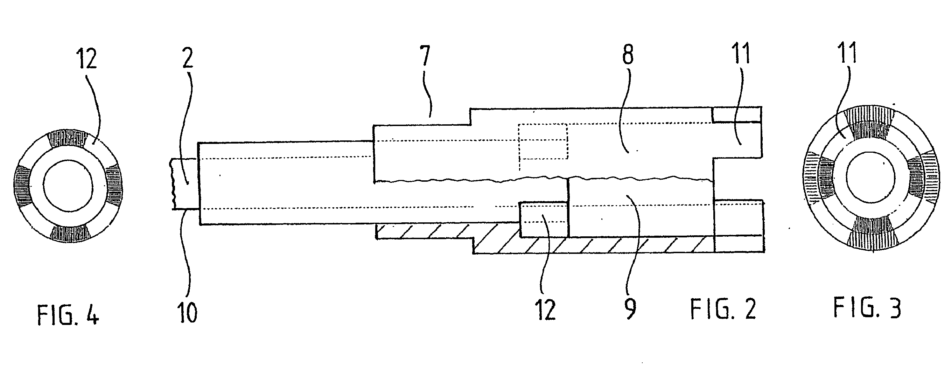

Die Figuren 2 bis 4 zeigen ebenfalls nur prinzipielle Funktionsbeispiele des Spann- und Verschlussmechanismus 7 bzw. dessen Schlüsselverzahnung 11 zum Ansetzen einer nicht dargestellten Montagehilfe und einen Schnitt durch eine Innenverrastung 12 von Innenbuchse 9 und Außenbuchse 8.

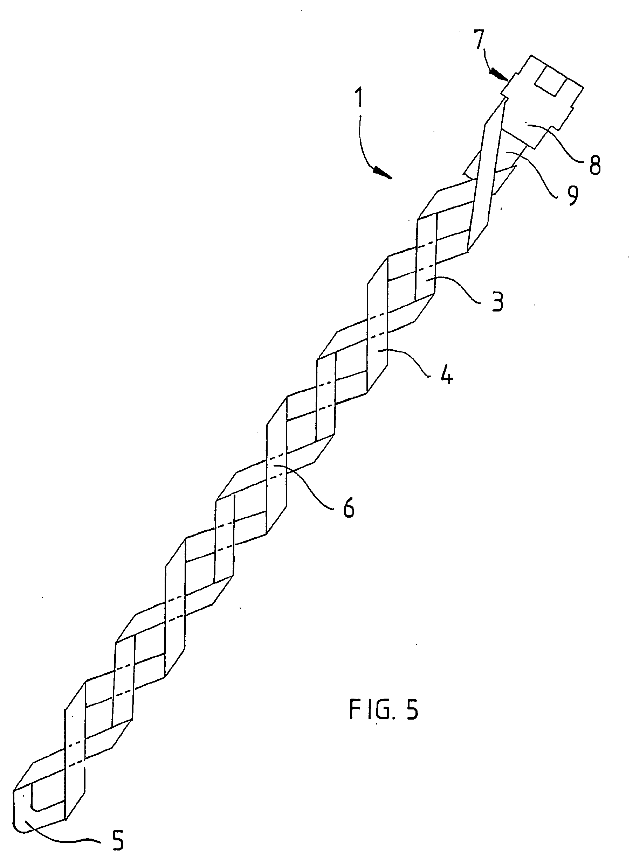

Die in Fig. 5 ebenfalls nur beispielhaft dargestellte Ausführungsform besitzt in einer praxisorientierten Ausführungsform ebenfalls eine deutlich höhere Anzahl von Windungen und hier nur ein einzelnes, von der als Kopfstück 5 ausgebildeten Spitze aus gegensinnig aufgewickeltes Band, dessen innere Wicklungen das inner Band 3 bilden und die äußeren Wicklungen das äußere Band 4. Der gegenständliche Nagelkern 2 ist hier bis auf der Spann- und Verschlussmechanismus 7 vollständig reduziert bzw. wird durch eine freien Luftraum ersetzt. Auch diese Version ermöglicht ein nach außen kräftefreies Verspannen des Marknagels, wie bei allen anderen vorgenannten Versionen auch, ohne dass mechanische Rückstellkräfte von einem Knochen aufgenommen oder in diesen eingeleitet werden müssten.

## Patentansprüche

1. Marknagel, insbesondere zur geschlossenen Reposition einer Fraktur eines Röhrenknochens durch Einsetzen in eine Bohrung in dessen Markhöhle, wobei der Marknagel (1) einen Nagelkern (2) aufweist, und ein Band (4), welches um den Nagelkern (2) herum aufgewickelt ist, **dadurch gekennzeichnet, dass** an vorderem Bereich des Marknagels (1) ein Kopfstück (5) vorgesehen ist, an dem der Anfang des Bandes (4) festgelegt ist, und das Band (4) an dessen Ende im hinteren Bereich um den Nagelkern (2) drehverriegelbar festgelegt ist und welches bei einer Drehung entgegen seiner Aufwickelrichtung die radiale Kontur des Marknagels (1) erweitert.

2. Marknagel nach Anspruch 1, **dadurch gekennzeichnet, dass** im vorderen Bereich des Nagelkerns (2) der Anfang eines weiteren inneren Bandes (3) festgelegt ist, welches unter dem Band (4) um den Nagelkern (2) herum aufgewickelt und dessen Ende im hinteren Bereich um den Nagelkern (2) drehverriegelbar festgelegt ist und welches sich bei einer Drehung entgegen dem Wickelsinn im Bereich von gemeinsamen Kontaktflächen (6) mit dem äußeren Band (4) an dieses anlegt und den Marknagel (1) stabilisiert.

3. Marknagel nach Anspruch 2, **dadurch gekennzeichnet, dass** die Bänder (3; 4) gleich- oder gegensinnig um den Nagelkern (2) herum aufgewickelt sind.

4. Marknagel nach Anspruch 2 oder 3, **dadurch gekennzeichnet, dass** zwei oder mehr Bänder (3; 4) im gleichen Wickelsinn mehrgängig um den Nagelkern (2) herum aufgewickelt sind.

5. Marknagel nach einem der Ansprüche 2 bis 4, **dadurch gekennzeichnet, dass** das innere Band (3) entlang seiner radialen Außenflächen und das äußere Band (4) entlang seiner radialen Innenflächen als gegenseitige Funktionsflächen ausgebildet sind, die als gemeinsame Kontaktflächen (6) Stabilisierungspunkte des in eine Durchgangsbohrung eines Knochens eingesetzten und verspannten Marknagels (1) in Form einer kraft- und reibschlüssigen Verbindung bilden.

6. Marknagel nach einem der Ansprüche 2 bis 5, **dadurch gekennzeichnet, dass** das äußere Band (4) und/ oder das innere Band (3) im unverbauten Zustand vorgespannt am Nagelkern (2) festgelegt sind/ ist oder dass deren hinteren Enden und das Ende des jeweils anderen Bandes (3; 4) gegeneinander verriegelt sind.

7. Marknagel nach einem der Ansprüche 2 bis 6, **dadurch gekennzeichnet, dass** das gegen den Wickelsinn abgewickeltes äußere Band (4) so ausgebildet ist, dass es sich an die Innenkonturen des Markraumes eines Röhrenknochens anlegt.

8. Marknagel nach einem der Ansprüche 2 bis 7, **dadurch gekennzeichnet, dass** das innere Band (3) und/ oder das äußere Band (4) in unterschiedlichen Bereichen des Nagelkerns (2) unterschiedlich breit und/ oder dick ausgebildet ist.

9. Marknagel nach einem der Ansprüche 2 bis 8, **dadurch gekennzeichnet, dass** das äußere Band (4) so ausgebildet ist, dass es beim Aufdrehen gegen den Wickelsinn sich zunächst im Bereich des Kopfstückes (5) des Marknagels (1) an die Innenkontur eines Knochens und bei weiterem Aufdrehen dann zunächst im mittleren Bereich eines Röhrenknochens und anschließend im Endbereich des Marknagels (1) anlegt.

10. Marknagel nach einem der Ansprüche 2 bis 9, **dadurch gekennzeichnet, dass** das innere Band (3) im Endbereich des Marknagels (1) an einer um den Nagelkern (2) drehbaren Innenbuchse (9) und dass äußere Band (4) an einer ebenfalls um den Nagelkern (2) drehbaren Außenbuchse (8) festgelegt ist und beide Buchsen (8;9) Rasten oder Rastflächen aufweisen, die über einen Spann- und Verschlussmechanismus gegeneinander und/ oder gegen den Nagelkern (2) verrastet oder verklemmt sind.

11. Marknagel nach Anspruch 10, **dadurch gekennzeichnet, dass** er eine davon lösbare Montagehilfe aufweist, die Betätigungsvorrichtungen für den Spann- und Verschlussmechanismus aufweist.

12. Marknagel nach einem der vorgenannten Ansprüche, **dadurch gekennzeichnet, dass** er an seinem vorderen oder hinteren Bereich Kontaktflächen zur Anordnung weiterer mechanischer Vorrichtungen zur Stabilisierung von Knochenbrüchen aufweist.

13. Marknagel nach einem der Ansprüche 10 bis 12, **dadurch gekennzeichnet, dass** das Ende des Nagelkerns (2) mit einem Gewinde (10) versehen ist, auf dem die Innenbuchse (9) verdrehbar aufgeschraubt ist.

14. Marknagel nach einem der Ansprüche 10 bis 13, **dadurch gekennzeichnet, dass** der Nagelkern (2) hohl ausgebildet ist und von einer Welle und/ oder Hülse durchtreten ist, an deren vorderen Ende der Anfang des Bandes (3, 4) festgelegt ist und dass am hinteren Ende des Marknagels (1) ein korrespondierender Spann- und Verschlussmechanismus angeordnet ist.

15. Marknagel nach Anspruch 14, **dadurch gekennzeichnet, dass** im vorderen Bereich des Nagelkerns (2) ein anderes äußeres Band (4) festgelegt ist als am hinteren Bereich des Marknagels (1) und dass beide Bänder (4) aufeinander zulaufen und in der gleichen Ebene in den jeweiligen Freiräumen des anderen Bandes sich bis zum jeweiligen anderen Ende des Nagelkerns (2) erstrecken.

16. Marknagel nach einem der vorgenannten Ansprüche, **dadurch gekennzeichnet, dass** er gerade oder gebogen ausgebildet ist.

17. Marknagel nach einem der vorgenannten Ansprüche, **dadurch gekennzeichnet, dass** der Nagelkern (2) mehrteilig ist und dass die Teile gegeneinander verdrehbar ausgebildet sind.

18. Marknagel nach einem der Ansprüche 2 bis 17, **dadurch gekennzeichnet, dass** zwei gegeneinander aufgewickelte Bänder (3; 4) an Ihren vorderen Enden miteinander verbunden sind oder dass ein Band einteilig ausgebildet und von seinem vorderen Ende aus gegensinnig um den Nagelkern (2) aufgewickelt ist und dass dieser Nagelkern (2) verkürzt ausgebildet ist und sich nur bis zu einem Teilbereich in Richtung des vorderen Endes des Marknagels (1) erstreckt.

19. Marknagel nach Anspruch 18, **dadurch gekennzeichnet, dass** der Nagelkern (2) bis auf den Spann- und Verschlussmechanismus (7) reduziert ist.

## Claims

1. Intramedullary nail, in particular for closed reduction of a fracture of a tubular bone by insertion into a bore in the medullary cavity thereof, the intramedullary nail (1) having a nail core (2), and a band (4) being wound on around the nail core (2), **characterized in that**, at the front area of the intramedullary nail (1), there is a head piece (5) on which the start of the band (4) is secured, and the band (4), at its end in the rear area, is secured so that it can be locked in rotation around the nail core (2) and which, in the event of a rotation counter to its direction of winding on, widens the radial contour of the intramedullary nail (1).

2. Intramedullary nail according to Claim 1, **characterized in that** the start of a further, inner band (3) is secured in the front area of the nail core (2), which further band (3) is wound on around the nail core (2) under the band (4) and its end, in the rear area, is secured so that it can be locked in rotation around the nail core (2) and which, in the event of a rotation counter to the direction of winding, bears on the outer band (4) in the region of common contact areas (6) with said outer band (4) and stabilizes the intramedullary nail (1).

3. Intramedullary nail according to Claim 2, **characterized in that** the bands (3; 4) are wound on around the nail core (2) in the same direction or in opposite directions.

4. Intramedullary nail according to Claim 2 or 3, **characterized in that** two or more bands (3; 4) are wound on around the nail core (2) in the same direction of winding in multiple turns.

5. Intramedullary nail according to one of Claims 2 to 4, **characterized in that** the inner band (3), along its radial outer surfaces, and the outer band (4), along its radial inner surfaces, are designed as mutual function surfaces which, at common contact areas (6), form stabilization points of the intramedullary nail (1) inserted into a through-bore of a bone and clamped, said stabilization points being established in the form of a frictional connection.

6. Intramedullary nail according to one of Claims 2 to 5, **characterized in that** the outer band (4) and/or the inner band (3) in the unworked state is/are secured with pretensioning on the nail core (2), or **in that** their rear ends and the end of the respective other band (3; 4) are locked against one another.

7. Intramedullary nail according to one of Claims 2 to 6, **characterized in that** the outer band (4) unwound counter to the direction of winding is designed such that it bears on the inner contours of the medullary space of a tubular bone.

8. Intramedullary nail according to one of Claims 2 to 7, **characterized in that** the inner band (3) and/or the outer band (4) are designed with a different width and/or a different thickness in different areas of the nail core (2).

9. Intramedullary nail according to one of Claims 2 to 8, **characterized in that** the outer band (4) is designed such that, when being turned counter to the direction of winding, it bears initially in the area of the head piece (5) of the intramedullary nail (1) on the inner contour of a bone and, upon further turning, then bears initially in the middle area of a tubular bone and thereafter in the end area of the intramedullary nail (1).

10. Intramedullary nail according to one of Claims 2 to 9, **characterized in that** the inner band (3) in the end area of the intramedullary nail (1) is secured on an inner bushing (9) which is rotatable about the nail core (2), and the outer band (4) is secured on an outer bushing (8) which is likewise rotatable about the nail core (2), and both bushings (8; 9) have locks or locking surfaces which are locked or clamped against one another and/or against the nail core (2) by a clamping and closing mechanism.

11. Intramedullary nail according to Claim 10, **characterized in that** it comprises a detachable assembly aid which has actuating devices for the clamping and closing mechanism.

12. Intramedullary nail according to one of the preceding claims, **characterized in that**, at its front or rear area, it has contact surfaces for the application of further mechanical devices for stabilization of bone fractures.

13. Intramedullary nail according to one of Claims 10 to 12, **characterized in that** the end of the nail core (2) is provided with a thread (10) on which the inner bushing (9) is screwed such that it can turn.

14. Intramedullary nail according to one of Claims 10 to 13, **characterized in that** the nail core (2) is hollow and is traversed by a shaft and/or sleeve at whose front end the start of the band (3; 4) is secured, and **in that** a corresponding clamping and closing mechanism is arranged at the rear end of the intramedullary nail (1).

15. Intramedullary nail according to Claim 14, **characterized in that**, in the front area of the nail core (2), a different outer band (4) is secured than at the rear area of the intramedullary nail (1), and **in that** both bands (4) run toward one another and extend in the same plane in the respective clearances of the other band as far as the respective other end of the nail core (2).

16. Intramedullary nail according to one of the preceding claims, **characterized in that** it is straight or bent.

17. Intramedullary nail according to one of the preceding claims, **characterized in that** the nail core (2) is in several parts and **in that** the parts are designed to be able to turn relative to one another.

18. Intramedullary nail according to one of Claims 2 to 17, **characterized in that** two bands (3; 4) wound on opposite to one another are connected to one another at their front ends, or **in that** a band is designed in one piece and, starting from its front end, is wound on in opposite directions around the nail core (2), and **in that** this nail core (2) is made short and extends only to a partial extent in the direction of the front end of the intramedullary nail (1).

19. Intramedullary nail according to Claim 18, **characterized in that** the nail core (2) is reduced to just the clamping and closing mechanism (7).

## Revendications

1. Clou médullaire, en particulier pour la réduction fermée d'une fracture d'un os long par insertion dans un alésage ménagé dans son creux médullaire, le clou médullaire (1) présentant une âme (2) de clou et un ruban (4) enroulé autour de l'âme (2) du clou,
**caractérisé en ce que**
une pièce de tête (5) à laquelle le début du ruban (4) est fixé est prévue dans la partie avant du clou médullaire (1), **en ce qu'**à son extrémité, le ruban (4) est fixé sans pouvoir tourner autour de l'âme (2) du clou dans sa partie arrière et en que lorsqu'il est tourné dans le sens contraire de son sens d'enroulement, il agrandit le contour radial du clou médullaire (1).

2. Clou médullaire selon la revendication 1, **caractérisé en ce que** le début d'un autre ruban intérieur (3) enroulé autour du clou médullaire (2) en dessous du ruban (4), dont l'extrémité est fixée de manière à ne pas pouvoir tourner autour de l'âme (2) du clou dans sa partie arrière tandis que lorsqu'il est tourné dans le sens opposé au sens d'enroulement, il repose sur le ruban extérieur (4) dans la zone des surfaces de contact (6) communes et stabilise le clou médullaire (1), est fixé dans la partie avant de l'âme (2) du clou.

3. Clou médullaire selon la revendication 2, **caractérisé en ce que** les rubans (3; 4) sont enroulés dans le même sens ou dans des sens opposés autour de l'âme (2) du clou.

4. Clou médullaire selon les revendications 2 ou 3, **caractérisé en ce que** deux ou plusieurs rubans (3; 4) sont enroulés plusieurs fois dans le même sens d'enroulement autour de l'âme (2) du clou.

5. Clou médullaire selon l'une des revendications 2 à 4, **caractérisé en ce que** la surface située radialement à l'extérieur du ruban intérieur (3) et la surface située radialement à l'intérieur du ruban extérieur (4) sont configurées comme surfaces fonctionnelles opposées qui, en tant que surfaces (6) de contact communes, forment par une liaison en correspondance mécanique et par frottement des points de stabilisation du clou médullaire (1) inséré et serré dans un alésage de passage ménagé dans l'os.

6. Clou médullaire selon l'une des revendications 2 à 5, **caractérisé en ce que** le ruban extérieur (4) et/ou le ruban intérieur (3) sont fixés avec précontrainte sur l'âme (2) du clou non monté et **en ce que** l'extrémité arrière de chaque ruban est verrouillée sur l'extrémité de l'autre ruban (3; 4).

7. Clou médullaire selon l'une des revendications 2 à 6, **caractérisé en ce que** le ruban extérieur (4) déroulé dans le sens opposé au sens d'enroulement est configuré de telle sorte qu'il repose sur le contour intérieur de l'espace médullaire d'un os long.

8. Clou médullaire selon l'une des revendications 2 à 7, **caractérisé en ce que** le ruban intérieur (3) et/ou le ruban extérieur (4) ont des largeurs et/ou des épaisseurs différentes dans différentes parties de l'âme (2) du clou.

9. Clou médullaire selon l'une des revendications 2 à 8, **caractérisé en ce que** le ruban extérieur (4) est configuré de telle sorte que lorsqu'il est tourné dans le sens opposé au sens d'enroulement, il repose d'abord dans la zone de la pièce de tête (5) du clou médullaire (1) sur le contour intérieur de l'os et lorsqu'il continue de tourner, il repose d'abord sur la partie centrale de l'os long et ensuite sur la partie terminale du clou médullaire (1).

10. Clou médullaire selon l'une des revendications 2 à 9, **caractérisé en ce que** dans la partie d'extrémité du clou médullaire (1), le ruban intérieur (3) est fixé sur une douille intérieure (9) qui peut tourner autour de l'âme (2) du clou et **en ce que** le ruban extérieur (4) est fixé sur une douille extérieure (8) qui peut également tourner autour de l'âme (2) du clou, les deux douilles (8; 9) présentant des encliquetages ou des surfaces d'encliquetage qui peuvent être encliquetées ou bloquées l'une sur l'autre et/ou sur l'âme (2) du clou par un mécanisme de serrage et de fermeture.

11. Clou médullaire selon la revendication 10, **caractérisé en ce qu'**il présente un accessoire de montage qui peut en être libéré et qui présente des dispositifs d'actionnement du mécanisme de serrage et de fermeture.

12. Clou médullaire selon l'une des revendications précédentes, **caractérisé en ce qu'**il présente sur sa partie avant ou sa partie arrière des surfaces de contact destinées à placer d'autres dispositifs mécaniques de stabilisation des fragments d'os.

13. Clou médullaire selon l'une des revendications 10 à 12, **caractérisé en ce que** l'extrémité de l'âme (2) du clou est dotée d'un filet (10) sur lequel la douille intérieure (9) est vissée de manière à pouvoir tourner.

14. Clou médullaire selon l'une des revendications 10 à 13, **caractérisé en ce que** l'âme (2) du clou est creuse et est traversée par un arbre et/ou une douille à l'extrémité avant desquels le début du ruban (3; 4) est fixé et **en ce qu'**un mécanisme correspondant de serrage et de fermeture est disposé à l'extrémité arrière du clou médullaire (1).

15. Clou médullaire selon la revendication 14, **caractérisé en ce que** dans la partie avant de l'âme (2) du clou est fixé un ruban extérieur (4) autre que dans la partie arrière du clou médullaire (1) et **en ce que** les deux rubans (4) se rapprochent l'un de l'autre et s'étendent dans le même plan dans les espaces libres respectifs de l'autre ruban jusqu'à l'autre extrémité respective de l'âme (2) du clou.

16. Clou médullaire selon l'une des revendications précédentes, **caractérisé en ce qu'**il est rectiligne ou cintré.

17. Clou médullaire selon l'une des revendications précédentes, **caractérisé en ce que** l'âme (2) du clou est réalisée en plusieurs pièces et **en ce que** les pièces peuvent tourner les unes par rapport aux autres.

18. Clou médullaire selon l'une des revendications 2 à 17, **caractérisé en ce que** deux rubans (3; 4) enroulés l'un sur l'autre sont reliés l'un à l'autre à leur extrémité avant ou **en ce qu'**un ruban est réalisé en une seule pièce et est enroulé dans des sens opposés autour de l'âme (2) du clou depuis son extrémité avant, et **en ce que** cette âme (2) de clou est plus courte et s'étend uniquement sur une partie en direction de l'extrémité avant du clou médullaire (1).

19. Clou médullaire selon la revendication 18, **caractérisé en ce que** l'âme (2) du clou est réduite jusqu'au mécanisme (7) de serrage et de fermeture.
